(19) Europäisches Patentamt / European Patent Office / Office européen des brevets

(11) **EP 4 173 646 B1**

(12) **EUROPEAN PATENT SPECIFICATION**

(45) Date of publication and mention
of the grant of the patent:
**01.10.2025 Bulletin 2025/40**

(21) Application number: **20951665.7**

(22) Date of filing: **14.10.2020**

(51) International Patent Classification (IPC):
**A61L 24/04** *(2006.01)*  **A61L 24/00** *(2006.01)*
**A61B 17/12** *(2006.01)*

(52) Cooperative Patent Classification (CPC):
(C-Sets available)
**A61B 17/12109; A61B 17/12031; A61B 17/1219;
A61L 24/0015; A61L 24/0042; A61L 24/046;**
A61B 2017/00004; A61L 2300/406    (Cont.)

(86) International application number:
**PCT/KR2020/013997**

(87) International publication number:
**WO 2022/045434 (03.03.2022 Gazette 2022/09)**

(54) **EMBOLIC MATERIAL PRODUCTION METHOD**

VERFAHREN ZUR HERSTELLUNG VON EMBOLISCHEM MATERIAL

PROCÉDÉ DE PRODUCTION DE MATÉRIAU EMBOLIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.08.2020 KR 20200106326**

(43) Date of publication of application:
**03.05.2023 Bulletin 2023/18**

(73) Proprietor: **Mepion Co., Ltd.
Seoul 08255 (KR)**

(72) Inventors:
• **KANG, Min Kyu
Seoul 04945 (KR)**
• **PARK, Sun Hee
Seoul 08745 (KR)**
• **NAM, Jung Hyuk
Seongnam-si Gyeonggi-do 13623 (KR)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
WO-A1-2020/116831    JP-B2- 4 442 302
JP-B2- 5 217 149    KR-A- 20170 009 700
KR-A- 20170 009 700    US-A1- 2019 192 438
US-A1- 2019 192 438

(52) Cooperative Patent Classification (CPC): (Cont.)

C-Sets
**A61L 24/046, C08L 67/04**

## Description

### Technical Field

The present disclosure relates to a method for producing an embolic material.

### Background Art

In general, embolization is known as a procedure that occludes a specific blood vessel by injecting an embolic material into the blood vessel. Since embolization can induce tumor necrosis by intentionally blocking a blood vessel supplying nutrients to the tumor, it has attracted attention as a safe and efficient surgical procedure that can replace surgical excision. In this regard, Korean Patent Application Publication No. 10-2015-0127135 proposes a technology related to an embolization system.

Currently, commercially available embolic materials are classified according to their form into particulate type, membrane type, and coil type. Among existing embolic materials, a particulate type embolic material does not biodegrade in blood vessels even after tumor necrosis, which may cause an inflammatory response. In addition, since the particulate type embolic material has poor elasticity or flexibility, it is inconvenient in that an operator should use the particulate type embolic material while taking into consideration the diameter of a blood vessel, the location and size of a lesion, and the like.

In addition, a membrane type embolic material needs to be cut to an appropriate size according to the size of a blood vessel and the type of lesion during a surgical procedure, and thus it is difficult to prepare an embolic material having a certain size and shape, and a sharp cut edge generated during the cutting process may cause damage to the inner wall of the blood vessel.

Furthermore, a coil-type embolic material has relatively high rigidity and sharpness compared to the particulate or membrane type embolic material, and thus it may cause emergency situations such as aneurysmal rupture during a surgical procedure. Furthermore, the process of removing the coil injected into the body must be performed after the embolization effect has been manifested, and thus this process acts as another burden on the operator and the patient.

Meanwhile, in the conventional art, when an embolic material is produced by a water-oil or oil-water emulsion method, it is not easy to produce beads having the size desired by the user, and thus it is difficult to obtain beads having a specific average particle diameter (e.g., 100 to 250 $\mu$m) in high yield.

In this regard, US 2019/192438 A1 discloses a method for preparing a degradable drug-loaded microsphere for embolization which includes dissolving a degradable material in an organic solvent, then adding a drug and mixing well to form a suspension or solution; then pouring the drug-containing suspension or solution into an aqueous solution of polyvinyl alcohol, stirring, and thereafter adding water twice for dilution, to prepare the degradable drug-loaded microsphere. KR 2017 0009700 A discloses a method of fabricating microparticles comprising a biodegradable polymer.

### Disclosure

### Technical Problem

The technical spirit of the present disclosure is to solve the above-described problems, and an object of the present disclosure is to provide a technology that is capable of producing an embolic material having a specific size in high yield.

Another object of the present disclosure is to provide an embolic material that induces blood vessel occlusion in a blood vessel and is naturally biodegradable after a certain period of time.

Still another object of the present disclosure is to provide an embolic material that is capable of minimizing damage to the inner wall of a blood vessel.

Yet another object of the present disclosure is to provide an embolic material that may be easily used by an operator without considering the diameter of a blood vessel, or the location or size of a lesion.

Still yet another object of the present disclosure is to provide a technology that is capable of easily adjusting the size of an embolic material during production of the embolic material.

### Technical Solution

The invention is set out in the independent claim. Preferred embodiments are reflected in the dependent claim.

To achieve the above objects, the present invention provides a method for producing an embolic material, the method including: a stirring step of mixing a biodegradable polymer solution and a surfactant solution at room temperature to obtain a stirred material; and a collection step of collecting beads from the stirred material; wherein in the stirring step, the surfactant solution contains multiple kinds of surfactants.

In addition, the surfactant solution is prepared by mixing 0.1 to 2 wt% of the plurality of surfactants and the balance of a solvent for the surfactants.

Furthermore, the plurality of surfactants includes methylcellulose and polyvinyl alcohol at a weight ratio of 1:1.

Furthermore, the biodegradable polymer solution is prepared by mixing a biodegradable polymer and a solvent for the polymer, and the biodegradable polymer is at least one selected from polycaprolactone and polylactic acid.

Furthermore, the biodegradable polymer solution is prepared by mixing 4 to 8 wt% of the biodegradable polymer and the balance of the solvent for the polymer.

In addition, the method for producing an embolic material may further include: a washing step of washing the beads; a drying step of drying the beads; a crosslinking step of crosslinking the beads; and a coating layer formation step of forming a coating layer on the surfaces of the beads.

**Advantageous Effects**

As described above, according to various embodiments of the present invention, it may be possible to produce beads having an average particle diameter of 100 to 250 $\mu$m in high yield.

In addition, it is possible to mix and stir the biodegradable polymer solution and the surfactant solution even at room temperature without increasing the stirring temperature, and thus there is an advantage in that process efficiency is improved.

Furthermore, according to various embodiments of the present invention, when the embolic material is injected into a blood vessel, the volume thereof may increase by self-expanding in the blood vessel, thus inducing blood vessel occlusion. In addition, as the volume of the embolic material increases by itself after injection into a blood vessel, the operator does not need to select and use an embolic material suitable for the diameter of the blood vessel, the location of a lesion, or the size of a lesion, and thus convenience in use is improved.

In addition, according to various embodiments of the present invention, the embolic material may be naturally removed by biodegradation in the blood vessel after a certain period of time, and thus a separate procedure for removing the embolic material is not required.

Furthermore, according to various embodiments of the present invention, the surface of the embolic material is a curved surface, and thus damage to the inner wall of a blood vessel by injection of the embolic material into the blood vessel may be minimized, thereby preventing an inflammatory response from being caused by damage to the inner wall.

Effects according to various embodiments of the present invention are not limited to the above-mentioned effects, and other effects not mentioned will be clearly understood by those skilled in the art from the following description.

**Description of Drawings**

FIG. 1 is a flowchart illustrating a method for producing an embolic material according to one embodiment of the present invention;

FIG. 2 is a graph showing the results obtained by measuring the time-dependent residual weight of an embolic material according to one embodiment of the present invention; and

FIG. 3 shows photographs of an embolic material according to one embodiment of the present invention that are taken with a metallographic microscope.

**Mode for Invention**

Preferred embodiments of the present invention will be described in more detail with reference to the accompanying drawings, but the descriptions of already known technical features will be omitted or simplified for the sake of brevity.

It should be noted that references to "an" or "one" embodiment of the invention in this specification are not necessarily directed to the same embodiment, and they imply at least one.

In the following embodiments, terms such as "first" and "second" are not used in a limited sense, but are used for the purpose of distinguishing one component from another component.

In the following examples, singular expressions include plural expressions unless the context clearly indicates otherwise.

In the following embodiments, terms such as "include" and "have" are intended to denote the presence of mentioned features or components, but do not exclude the probability of presence or addition of one or more other features or components.

When a certain embodiment may be implemented differently, a particular process order may be performed differently from the described order. For example, two consecutively described processes may be performed substantially at the same time or performed in the order opposite to the described order. That is, each step of the method described herein may be suitably performed in any order, unless otherwise indicated herein or clearly contradicted by context.

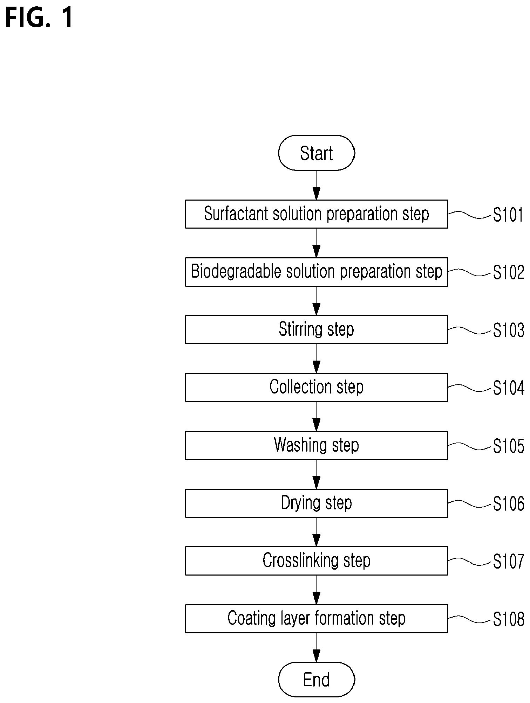

A method for producing an embolic material according to one embodiment of the present invention will be described according to the flowchart shown in FIG. 1, will be described with reference to the remaining drawings, and will be described in a stepwise manner for convenience of description.

1. Surfactant solution preparation step <S101>

In this step, a process of preparing a surfactant solution by mixing multiple kinds of surfactants and a solvent for the surfactants is performed. The plurality of surfactants includes methylcellulose as a first surfactant and polyvinyl alcohol as a second surfactant. In addition, as the solvent for the surfactants according to one embodiment, water, distilled water, or water for injection may be used.

In this step, the surfactant solution is prepared by mixing 0.1 to 2 wt% of the plurality of surfactants and the balance of the solvent for the surfactants. In this case, "0.1 to 2 wt% of the plurality of surfactants" means the total weight of the mixture of methylcellulose as the first surfactant and polyvinyl alcohol as the second surfactant.

As a specific example, the content of the plurality of surfactants may be 0.1 wt%, 0.2 wt%, 0.3 wt%, 0.4 wt%, 0.5 wt%, 0.6 wt%, 0.7 wt%, 0.8 wt%, 0.9 wt%, 1 wt% %, 1.1 wt%, 1.2 wt%, 1.3 wt%, 1.4 wt%, 1.5 wt%, 1.6 wt%, 1.7 wt%, 1.8 wt%, 1.9 wt%, or 2 wt%. In addition, the content of the plurality of surfactants may range from a value equal to or greater than any one of the above values to a value equal to or smaller than any one of the above values.

For example, the content range of the plurality of surfactants may be 0.1 wt% to 1 wt%, 0.5 wt% to 1.5 wt%, 1 wt% to 2 wt%, or 0.1 wt% to 2 wt%. According to one embodiment, when the content of the plurality of surfactants is within the above range, it is easy to produce the embolic material.

If the content of the plurality of surfactants is less than 0.1 wt% or more than 2 wt%, the productivity of the embolic material beads may decrease and the yield may decrease. For this reason, the content of the plurality of surfactants is preferably within the above-described range.

Methylcellulose and polyvinyl alcohol in the content of the plurality of surfactants is applied at a weight ratio of 1:1.

In this step, the surfactant solution may be prepared by mixing 0.1 to 2 wt% of the plurality of surfactants and 98 to 99.9 wt% of a solvent for the surfactants so that the surfactants may be sufficiently dissolved in the solvent, and stirring the mixture at 200 to 500 rpm for 3 to 5 hours. It is obvious that in this step, the stirring speed and the stirring time may be vary depending on experimental conditions.

2. Biodegradable polymer solution preparation step <S102>

In this step, a biodegradable polymer solution is prepared by mixing a biodegradable polymer and a solvent for the polymer. The biodegradable polymer is at least one selected from polycaprolactone and polylactic acid. In one embodiment, the solvent for the polymer is a solvent capable of dissolving the biodegradable polymer, and non-limiting examples of the solvent for the polymer include chloroform.

In this step, the biodegradable polymer solution is prepared by mixing 4 to 8 wt% of the biodegradable polymer and the balance of the solvent for the polymer. As a specific example, the content of the biodegradable polymer may be 4 wt%, 5 wt%, 6 wt%, 7 wt%, or 8 wt%. In addition, the content of the biodegradable polymer may be may range from a value equal to or greater than any one of the above values to a value equal to or smaller than any one of the above values.

For example, the content range of the biodegradable polymer may be 4 wt% to 6 wt%, 5 wt% to 7 wt%, 6 wt% to 8 wt%, or 4 wt% to 8 wt%. According to one embodiment, when the content of the biodegradable polymer is within the above range, it is easy to produce the embolic material.

If the content of the biodegradable polymer is less than 4 wt%, a problem may arise in that the size of the embolic material beads produced is excessively small, and thus it is difficult to collect the beads, and if the content of the biodegradable polymer is more than 8 wt%, a problem may arise in that the beads stick together or agglomerate, making it difficult to produce beads having a desired size (or a desired size). For these reasons, the content of the biodegradable polymer is preferably within the above-described range.

In this step, 4 to 8 wt% of the biodegradable polymer is mixed with 92 to 96 wt% of the solvent for polymer so that the biodegradable polymer may be sufficiently dissolved in the solvent, and the mixture is stirred at 200 to 500 rpm for 3 to 5 hours at a temperature of 45 to 60°C. It is obvious that in this step, the stirring speed, stirring time, and stirring temperature may vary according to experimental conditions.

Meanwhile, the step of preparing the biodegradable polymer solution and the step of preparing the surfactant solution may be performed at the same time. Alternatively, the step of preparing the biodegradable polymer solution may be performed before the step of preparing the surfactant solution. Alternatively, the step of preparing the biodegradable polymer solution may be performed after the step of preparing the surfactant solution.

### 3. Stirring step <S103>

In this step, the biodegradable polymer solution and the surfactant solution is mixed together at room temperature to obtain a stirred material. As used herein, the term "room temperature" is a natural temperature without heating or cooling, which may be, for example, 20 to 25°C.

The surfactant solution that is used in this step contains methylcellulose as a first surfactant and polyvinyl alcohol as a second surfactant at a weight ratio of 1:1.

In this step, the biodegradable polymer solution may be poured into a container containing the surfactant solution and stirred at a high speed of 250 to 1000 rpm. In addition, during stirring, the solvent for the polymer, contained in the biodegradable polymer solution, may be volatilized using an exhaust ventilation system. Stirring and volatilization of the solvent for the polymer may be performed simultaneously and may be performed for 10 to 12 hours.

In this step, stirring may be performed smoothly at room temperature without raising the temperature by heating the stirred material. Through this step, the embolic material may be cured and produced, and the embolic material produced may have a spherical shape with a curved surface, and thus it is possible to prevent or minimize the inner wall from being damaged when the inner wall of a blood vessel comes into contact with the embolic material.

### 4. Collection step <S104>

In this step, a process of collecting beads from the stirred material obtained in step S103 is performed. As a specific example, in this step, beads included in the stirred material may be filtered through a 0.2 $\mu$m filter, and only beads may be selectively collected from the stirred material.

### 5. Washing step <S105>

In this step, the beads collected in step S104 may be washed. For example, the beads may be washed by setting the collected polymer beads and water for injection to s weight ratio of 1:5 and mixing them. In this step, the surfactant component remaining on the surface or inside of the bead may be removed by washing. After completion of washing in this step, the beads may be collected through a 0.2 $\mu$m filter. In addition, it is also possible to repeat this step three times or more, if necessary.

### 6. Drying step <S106>

In this step, the beads washed in step S105 may be dried. For example, the beads may be dried using a vacuum oven or a freeze dryer. When the beads are dried using a vacuum oven, the beads may be dried for 48 hours at a vacuum level of 1 mTorr and an oven temperature of 35°C. When the beads are dried using a freeze dryer, the beads may be frozen at -40°C for 2 hours or more, and then sublimated while rising to 4°C. It is obvious that the beads may be dried using various known drying methods and drying conditions other than the above-described examples.

### 7. Crosslinking step <S107>

In this step, a process of crosslinking the beads dried in step S106 may be performed. As a specific example, in this step, the beads may be crosslinked by a physical, chemical or enzymatic crosslinking method. For example, the beads may be crosslinked by at least one crosslinking method selected from the group consisting of ultraviolet light irradiation, irradiation with radiation, heat treatment, high-temperature dehydrothermal treatment (DHT), 1-ethyl-3-(3-dimethylaminopropyl)carbodiimide (EDC) treatment, N-hydroxysuccinimide (NHS) treatment, and glutaraldehyde treatment, but the method of crosslinking the beads is not limited only to the above examples, and other known crosslinking methods may also be applied to crosslink the biodegradable polymer beads. As a more specific example, when the beads are crosslinked by high-temperature dehydrothermal treatment, the beads may be crosslinked by putting the beads in an oven and allowing the beads to react at a temperature of 100 to 200°C for 24 to 36 hours under a pressure of 0.01 to 0.1 bar. In addition, when the beads are crosslinked by EDC treatment, the beads may be immersed in a solution, obtained by dissolving EDC in 95 v/v% ethanol at a concentration of 0.1 to 100 mM, and then crosslinked by keeping in the solution at 2 to 4°C for 24 to 48 hours, and then excess EDC may be washed out with purified water, thus producing crosslinked beads.

The reaction conditions (pressure, temperature, time, etc.) during the above-described high-temperature dehydrothermal treatment or the reaction conditions (e.g., concentration of EDC, time, temperature, etc.) during EDC treatment may be flexibly changed by a person skilled in the art depending on the degree of crosslinking reaction of the beads. Furthermore, specific crosslinking methods for crosslinking biodegradable polymers are well-known techniques in the art, and thus further detailed descriptions thereof are omitted herein.

In addition, since this step is performed to control the *in vivo* biodegradation rate of the beads, this step may also be omitted depending on the implementation.

8. Coating layer formation step <S108>

In this step, a coating layer may be formed on the surface of the bead. In one embodiment, a coating layer may be formed on the surface of the bead by spraying a coating solution onto the surface of the bead. It is obvious that depending on the implementation, it is also possible to form a coating layer on the surface of the bead by immersing the bead in a container containing a coating solution, and various other coating methods may be applied. In one embodiment, the coating solution refers to a solution obtained by mixing a coating material and a solvent for the coating material, and non-limiting examples of a coating material that may be applied in this step include gentamicin, hyaluronic acid, collagen, and the like. In addition, depending on the implementation, the coating material may be a combination of two or more materials mixed together. As an example of the combination as the coating solution, a coating solution which is a mixture of water, gentamicin and hyaluronic acid may be used.

The concentration of the coating solution according to one embodiment may be 0.5 to 1 w/w%, but may be variously changed depending on the types of coating material and solvent.

Hereinafter, the present invention will be described in more detail with reference to specific examples. The following examples are only examples intended to assist in understanding of the present invention, so that the scope of the present invention is not limited or restricted thereto.

Production of embolic materials

<Examples 1 to 5 and Comparative Examples 1 and 2>

Water for injection, methylcellulose, and polyvinyl alcohol were mixed together in the amounts (unit: g) shown in Table 1 below, and stirred at 200 to 500 rpm for 3 to 5 hours, thus preparing a surfactant solution. In addition, 8 g of a biodegradable polymer (polycaprolactone) was mixed with 92 g of chloroform and stirred at 200 to 500 rpm for 3 to 5 hours at a stirring temperature of 45 to 60°C, thus preparing a biodegradable polymer solution. Thereafter, the biodegradable polymer solution was poured into the prepared surfactant solution and stirred at 250 to 1,000 rpm at room temperature for 10 to 12 hours, and chloroform was volatilized using an exhaust ventilation system during stirring. Thereafter, the beads included in the stirred material were collected through a 0.2 μm filter, and the collected beads were washed by mixing the beads and water for injection at a weight ratio of 1:5. Washing of the beads was repeated three times. The washed beads were placed in a vacuum oven and dried at a vacuum level of 1 mTorr and an oven temperature of 35°C for 48 hours. Next, the dried beads were immersed in 100 ml of a solution, obtained by dissolving EDC in 95 v/v% ethanol at a concentration of 10 mM, and crosslinked by keeping in the solution at 2 to 4°C for 24 to 48 hours. The crosslinked beads were washed with 500 to 1,000 ml of distilled water at room temperature for 60 minutes, washed 6 times, and dried by natural ventilation for 1 to 3 days. Thereafter, a solution containing gentamicin at a concentration of 1 w/w% was sprayed on the surface of the bead through a fluidized bed dryer to form a coating layer on the surface of the bead, thereby producing embolic materials. The fluidized bed dryer was operated under the conditions of fluidized bed inlet air temperature of 100°C, product temperature of 70°C, exhaust damper of 30%, fan inverter of 70%, coating solution spray pump RPM of 3, and air pressure of 1.5K during coating solution spray.

[Table 1]

|  | Example 1 | Example 2 | Example 3 | Example 4 | Example 5 | Comparative Example 1 | Comparative Example 2 |
|---|---|---|---|---|---|---|---|
| Methylcellulose | 0.05 | 0.25 | 0.5 | 0.75 | 1 | 0.05 | 1.5 |
| Polyvinyl alcohol | 0.05 | 0.25 | 0.5 | 0.75 | 1 | 0 | 1.5 |
| Water for injection | 99.9 | 99.5 | 99 | 98.5 | 98 | 99.95 | 97 |

<Examples 6 to 10>

Water for injection, methylcellulose, and polyvinyl alcohol were mixed together in the amounts (unit: g) shown in Table 2 below, and stirred at 200 to 500 rpm for 3 to 5 hours, thus preparing a surfactant solution. In addition, 8 g of a biodegradable polymer (poly-L-lactic acid (PLLA)) was mixed with 92 g of chloroform and stirred at 200 to 500 rpm for 3 to 5 hours at a stirring temperature of 45 to 60°C, thus preparing a biodegradable polymer solution. Thereafter, the biodegradable polymer solution was poured into the prepared surfactant solution and stirred at 250 to 1,000 rpm at room temperature for 10 to 12 hours, and chloroform was volatilized using an exhaust ventilation system during stirring. Thereafter, the beads included in the stirred material were collected through a 0.2 μm filter, and the collected beads were washed by mixing the beads and water for injection at a weight ratio of 1:5. Washing of the beads was repeated three times. The washed beads were placed in a vacuum oven and dried at a vacuum level of 1 mTorr and an oven temperature of 35°C for 48 hours. Next, the dried beads were immersed in 100 ml of a solution, obtained by dissolving EDC in 95 v/v% ethanol at a concentration of 10 mM, and crosslinked by keeping in the solution at 2 to 4°C for 24 to 48 hours. The crosslinked beads were washed with 500 to 1,000 ml of distilled water at room temperature for 60 minutes, washed 6 times, and dried by natural ventilation for 1 to 3 days. Thereafter, a solution containing gentamicin at a concentration of 1 w/w% was sprayed on the surface of the bead through a fluidized bed dryer to form a coating layer on the surface of the bead, thereby producing embolic materials. The fluidized bed dryer was operated under the conditions of fluidized bed inlet air temperature of 100°C, product temperature of 70°C, exhaust damper of 30%, fan inverter of 70%, coating solution spray pump RPM of 3, and air pressure of 1.5K during coating solution spray.

[Table 2]

|  | Example 6 | Example 7 | Example 8 | Example 9 | Example 10 |
|---|---|---|---|---|---|
| Methylcellulose | 0.05 | 0.25 | 0.5 | 0.75 | 1 |
| Polyvinyl alcohol | 0.05 | 0.25 | 0.5 | 0.75 | 1 |
| Water for injection | 99.9 | 99.5 | 99 | 98.5 | 98 |

<Examples 11 to 15 and Comparative Examples 3 and 4>

Embolic materials were produced in the same manner as in Example 2, except that a biodegradable polymer solution was prepared by mixing a biodegradable polymer (polycaprolactone) and chloroform in the amounts (unit: g) shown in Table 3 below and stirring the mixture at 200 to 500 rpm for 3 to 5 hours at a stirring temperature of 45 to 60°C.

[Table 3]

|  | Example 11 | Example 12 | Example 13 | Example 14 | Example 15 | Comparative Example 3 | Comparative Example 4 |
|---|---|---|---|---|---|---|---|
| Polycaprolactone | 4 | 5 | 6 | 7 | 8 | 3 | 9 |
| Chloroform | 96 | 95 | 94 | 93 | 92 | 97 | 91 |

<Examples 16 to 20 and Comparative Examples 5 and 6>

Embolic materials were produced in the same manner as in Example 2, except that a biodegradable polymer solution was prepared by mixing a biodegradable polymer (poly-L-lactic acid) and chloroform in the amounts (unit: g) shown in Table 4 below and stirring the mixture at 200 to 500 rpm for 3 to 5 hours at a stirring temperature of 45 to 60°C.

[Table 4]

|  | Example 16 | Example 17 | Example 18 | Example 19 | Example 20 | Comparative Example 5 | Comparative Example 6 |
|---|---|---|---|---|---|---|---|
| PLLA | 4 | 5 | 6 | 7 | 8 | 3 | 9 |
| Chloroform | 96 | 95 | 94 | 93 | 92 | 97 | 91 |

<Example 21 and Comparative Examples 7 to 10>

Embolic materials were produced in the same manner as in Example 2, except that a surfactant solution was prepared by mixing water for injection, methylcellulose and polyvinyl alcohol in the amounts (unit: g) shown in Table 5 below and stirring the mixture at 200 to 500 rpm for 3 to 5 hours.

[Table 5]

|  | Example 21 | Comparative Example 7 | Comparative Example 8 | Comparative Example 9 | Comparative Example 10 |
|---|---|---|---|---|---|
| Methylcellulose | 0.25 | 0 | 0.5 | 0.25 | 0.25 |
| Polyvinyl alcohol | 0.25 | 0.25 | 0.25 | 0.5 | 0 |
| Water for injection | 99.5 | 99.75 | 99.25 | 99.25 | 99.75 |

Evaluation of productivity of embolic material

The embolic material beads produced in each of the Example and the Comparative Example are classified by size using a sieve, and evaluation was performed based on the following criteria. Excellent productivity: the proportion of beads having an average particle diameter of 125 to 250 $\mu$m among the total beads produced is 70% or more; and poor productivity: the proportion is less than 70%. The results are shown in Table 6, Table 7, Table 8, and Table 9 below.

[Table 6]

| | Example 1 | Example 2 | Example 3 | Example 4 | Example 5 | Example 6 | Example 7 | Example 8 | Example 9 | Example 10 | Comp. Example 1 | Comp. Example 2 |
|---|---|---|---|---|---|---|---|---|---|---|---|---|
| Productivity | Excellent | Excellent | Excellent | Excellent | Excellent | Excellent | Excellent | Excellent | Excellent | Excellent | Poor | Poor |

As shown in Table 6 above, it can be confirmed that, in Examples 1 to 10, the proportion of beads having an average particle diameter of 125 to 250 μm was 70% or more, and thus the productivity for the embolic material having a specific size was excellent. In addition, from the experimental results of Comparative Examples 1 and 2, it can be confirmed that, when the contents of the plurality of surfactants were out of the predetermined ranges, the productivity of the embolic material having a specific size was poor.

[Table 7]

|  | Example 11 | Example 12 | Example 13 | Example 14 | Example 15 | Comparative Example 3 | Comparative Example 4 |
|---|---|---|---|---|---|---|---|
| Productivity | Excellent | Excellent | Excellent | Excellent | Excellent | Poor | Poor |

As shown in Table 7 above, it was confirmed that, in Examples 11 to 15, the proportion of beads having an average particle diameter of 125 to 250 μm was 70% or more, and thus the productivity for the embolic material having a specific size was excellent, whereas, in Comparative Example 3, productivity was poor because the size of the beads was excessively small, and in Comparative Example 4, productivity was poor because agglomeration occurred while the beads stuck together during the stirring step.

[Table 8]

|  | Example 16 | Example 17 | Example 18 | Example 19 | Example 20 | Comparative Example 5 | Comparative Example 6 |
|---|---|---|---|---|---|---|---|
| Productivity | Excellent | Excellent | Excellent | Excellent | Excellent | Poor | Poor |

As shown in Table 8 above, it was confirmed that, in Examples 16 to 20, the proportion of beads having an average particle diameter of 125 to 250 μm was 70% or more, and thus the productivity for the embolic material having a specific size was excellent, whereas, in Comparative Example 5, productivity was poor because the size of the beads was excessively small, and in Comparative Example 4, productivity was poor because agglomeration occurred while the beads stuck together during the stirring step.

[Table 9]

|  | Example 21 | Comparative Example 7 | Comparative Example 8 | Comparative Example 9 | Comparative Example 10 |
|---|---|---|---|---|---|
| Productivity | Excellent | Poor | Poor | Poor | Poor |

As shown in Table 9, it can be seen that, in Example 21, the proportion of beads having an average particle diameter of 125 to 250 μm was 70% or more, and thus the productivity for the embolic material having a specific size was excellent. In addition, from the experimental results of Comparative Examples 7 to 10, it can be confirmed that the productivity for the embolic material having a specific size was poor when the weight ratio of the first surfactant to the second surfactant was not appropriate.

Evaluation of time-dependent biodegradability of embolic material

In order to establish the expiration date and *in vitro* biodegradability of the embolic material, an accelerated aging test was conducted according to the stability test standards for medical devices (Ministry of Food and Drug Safety Notice No. 2016-70) and ASTM F 1980. In order to evaluate the time-dependent biodegradability of the embolic material according to Example 2, the change in the weight of the embolic material was measured for a total of 24 months. The sample was stored in a 5-ml glass vial with 5 ml of a degradation solvent (physiological saline). The sample was stored in a constant temperature and humidity chamber at 60% humidity and 50°C, and collected on days corresponding to 0.5, 1, 2, 3, 4, 5, 7, 8, 10, 12, 18, and 24 months for calculation of the accelerated aging period, and the embolic material was collected therefrom, washed with purified water, dried, and then weighed. The biodegradability of the embolic material was evaluated by expressing the residual weight of the embolic material at each time point as a percentage.

• Calculation of accelerated aging test period (based on 24 months (2 years))

$$AAF = \text{accelerated aging factor} = Q_{10}^{[(TAA-TRT)/10]}$$

TAA ≡ accelerated aging temperature (°C) = 50°C
TRT ≡ ambient temperature (°C) = 22°C

$$(\therefore) \quad AAF = Q_{10}^{[(TAA-TRT)/10]} = 2^{[(50-22)/10]} = 6.96 \quad (Q_{10}=2)$$

Accelerated aging temperature (AAT)= set (RT)/AAF = 730 days (2 years)/6.96 = 104.89 ≒ 105 (days)

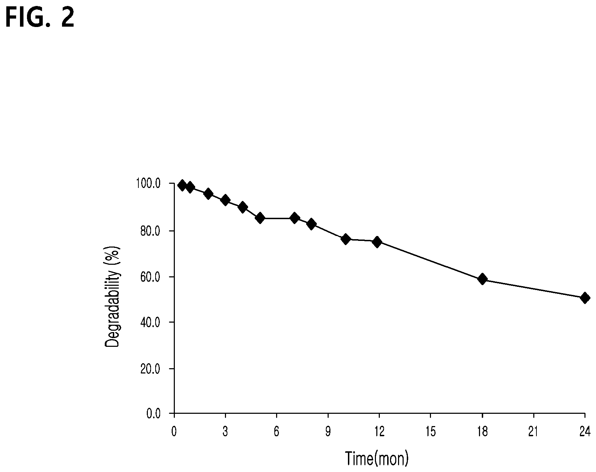

FIG. 2 is a graph showing the results obtained by measuring the time-dependent residual weight of the embolic material according to Example 2 of the present invention. Referring to FIG. 2, it can be seen that the biodegradation of the embolic material proceeded gradually over time.

Measurement of time-dependent self-expanding ability of embolic material

The change in the volume of the embolic material according to Example 2 was measured using a metallurgical microscope (DSX 510 from OLYMPUS). Specifically, the embolic material was immersed in phosphate buffered saline (PBS) at intervals of 30 minutes, 1 hour, 2 hours, and 24 hours, and then the surface area of the sample with increased volume was measured. The results are shown in Table 10 below.

[Table 10]

| Time (hr) | Embolic material area ($\mu m^2$) | Increase rate (% of initial area) |
| --- | --- | --- |
| 0 | 7,508.113 | 0 |
| 0.5 | 12,651.505 | 68.50 |
| 1 | 35,300.851 | 370.17 |
| 2 | 40,164.207 | 434.94 |
| 24 | 63,979.866 | 752.14 |

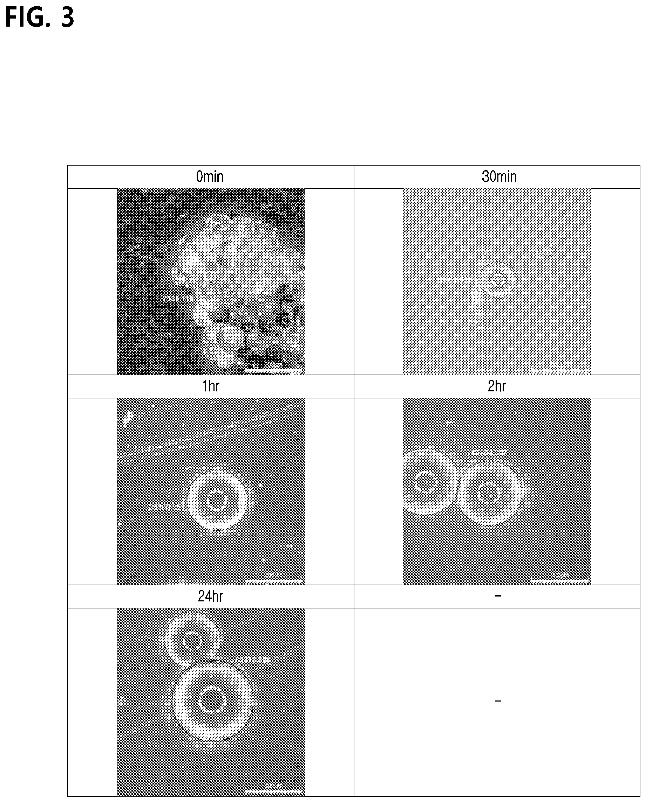

As shown in Table 10 above, it can be confirmed that the diameter of the embolic material according to Example 2 increased by reaction with phosphate-buffered saline, which is a condition similar to bodily fluid, for a certain period of time, suggesting that when it is injected into a blood vessel, it can sufficiently function as an embolic material that induces blood vessel occlusion. FIG. 3 shows photographs taken with a metallographic microscope for the change in the volume of the embolic material according to Example 2 of the present invention after immersing the embolic material in phosphate buffered saline (PBS) at intervals of 30 minutes, 1 hour, 2 hours, and 24 hours.

Referring to FIG. 3, it can be seen that the area of the embolic material increased over time. In addition, since the embolic material produced may have a spherical shape with a curved surface, it is possible to prevent or minimize the inner wall of a blood vessel from being damaged when the inner wall of the blood vessel comes into contact with the embolic material.

As described above, according to various embodiments of the present invention, it is possible to produce beads having an average particle diameter of 125 to 250 $\mu$m, which is a size widely used as an embolic material, in high yield. Therefore, the process of sorting the produced embolic beads by size is simplified, and thus the efficiency and productivity of the production process are improved.

In addition, by specifying the weight ratio of the plurality of surfactants and specifying the content of the biodegradable polymer, it is possible to mix and stir the biodegradable polymer solution and the surfactant solution even at room temperature without increasing the stirring temperature, and thus process efficiency and workability are improved.

Furthermore, according to various embodiments of the present invention, when the embolic material is injected into a blood vessel, the volume thereof may increase by self-expanding in the blood vessel, thus inducing blood vessel occlusion. In addition, as the volume of the embolic material increases by itself after injection into a blood vessel, the operator does not need to select and use an embolic material suitable for the diameter of the blood vessel, the location of a lesion, or the size of a lesion, and thus convenience in use is improved.

In addition, according to various embodiments of the present invention, the embolic material may be naturally removed by biodegradation in the blood vessel after a certain period of time, and thus a separate procedure for removing the embolic material is not required.

In addition, according to various embodiments of the present invention, the surface of the embolic material is a curved surface, and thus damage to the inner wall of a blood vessel by injection of the embolic material into the blood vessel may be minimized, thereby preventing an inflammatory response from being caused by damage to the inner wall.

**Claims**

1. A method for producing an embolic material, the method comprising:

a stirring step of mixing a biodegradable polymer solution and a surfactant solution in a weight ratio of 1:1 at room temperature to obtain a stirred material; and
a collection step of collecting beads from the stirred material;
wherein in the stirring step, the surfactant solution contains multiple kinds of surfactants,
**characterized in that**,
the surfactant solution is prepared by mixing 0.1 to 2 wt% of the plurality of surfactants and the balance of a solvent for the surfactants,
the plurality of surfactants comprises methylcellulose and polyvinyl alcohol at a weight ratio of 1:1,
the biodegradable polymer solution is prepared by mixing a biodegradable polymer and a solvent for the polymer, and the biodegradable polymer is at least one selected from polycaprolactone and polylactic acid, and
the biodegradable polymer solution is prepared by mixing 4 to 8 wt% of the biodegradable polymer and the balance of the solvent for the polymer.

2. The method according to claim 1, further comprising:

a washing step of washing the beads;
a drying step of drying the beads;
a crosslinking step of crosslinking the beads; and
a coating layer formation step of forming a coating layer on surfaces of the beads.

**Patentansprüche**

1. Verfahren zur Herstellung eines embolischen Materials, wobei das Verfahren umfasst:

einen Rührschritt, bei dem eine Lösung eines bioabbaubaren Polymers und eine Tensidlösung in einem Gewichtsverhältnis von 1:1 bei Raumtemperatur gemischt werden, wobei man ein gerührtes Material erhält; und
einen Auffangschritt, bei dem Beads aus dem gerührten Material aufgefangen werden;
wobei in dem Rührschritt die Tensidlösung mehrere Arten von Tensiden enthält,
**dadurch gekennzeichnet, dass**
die Tensidlösung dadurch hergestellt wird, dass man 0,1 bis 2 Gew.-% der Vielzahl von Tensiden und als Rest ein Lösungsmittel für die Tenside miteinander mischt,
die Vielzahl von Tensiden Methylcellulose und Polyvinylalkohol in einem Gewichtsverhältnis von 1:1 umfasst,
die Lösung des bioabbaubaren Polymers dadurch hergestellt wird, dass man ein bioabbaubares Polymer und ein Lösungsmittel für das Polymer miteinander mischt, und das bioabbaubare Polymer wenigstens eines ist, das aus Polycaprolacton und Polymilchsäure ausgewählt ist, und
die Lösung des bioabbaubaren Polymers dadurch hergestellt wird, dass man 4 bis 8 Gew.-% des bioabbaubaren Polymers und als Rest das Lösungsmittel für das Polymer miteinander mischt.

2. Verfahren gemäß Anspruch 1, weiterhin umfassend:

einen Waschschritt, bei dem die Beads gewaschen werden;
einen Trockenschritt, bei dem die Beads getrocknet werden;
einen Vernetzungsschritt, bei dem die Beads vernetzt werden; und
einen Beschichtungsschicht-Bildungsschritt, bei dem auf Oberflächen der Beads eine Beschichtungsschicht gebildet wird.

**Revendications**

1. Procédé de production d'un matériau embolique, le procédé comprenant :

une étape d'agitation consistant à mélanger une solution de polymère biodégradable et une solution de tensioactif dans un rapport pondéral de 1 : 1 à température ambiante pour obtenir un matériau agité ; et

une étape de prélèvement consistant à prélever des billes à partir du matériau agité ;

dans lequel, dans l'étape d'agitation, la solution de tensioactif contient plusieurs types de tensioactifs, **caractérisé en ce que**

la solution de tensioactif est préparée en mélangeant 0,1 à 2 % en poids de la pluralité de tensioactifs et le reste d'un solvant pour les tensioactifs,

la pluralité de tensioactifs comprend de la méthylcellulose et de l'alcool polyvinylique dans un rapport pondéral de 1 : 1,

la solution de polymère biodégradable est préparée en mélangeant un polymère biodégradable et un solvant pour le polymère, et le polymère biodégradable est au moins un polymère choisi parmi la polycaprolactone et l'acide polylactique, et

la solution de polymère biodégradable est préparée en mélangeant 4 à 8 % en poids du polymère biodégradable et le reste du solvant pour le polymère.

2. Procédé selon la revendication 1, comprenant en outre :

une étape de lavage consistant à laver les billes ;
une étape de séchage consistant à sécher les billes ;
une étape de réticulation consistant à réticuler les billes ; et
une étape de formation de couche de revêtement consistant à former une couche de revêtement sur des surfaces des billes.

# FIG. 1

Start

Surfactant solution preparation step — S101

Biodegradable solution preparation step — S102

Stirring step — S103

Collection step — S104

Washing step — S105

Drying step — S106

Crosslinking step — S107

Coating layer formation step — S108

End

# FIG. 2

# FIG. 3

| 0min | 30min |
|------|-------|
| | |
| **1hr** | **2hr** |
| | |
| **24hr** | – |
| | – |

**EP 4 173 646 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020150127135
- US 2019192438 A1
- KR 20170009700 A